# EUROPEAN PATENT SPECIFICATION

(11) **EP 2 674 148 B1**
(45) Date of publication and mention of the grant of the patent: **01.02.2017**
(21) Application number: 11857980.4
(22) Date of filing: 19.12.2011
(51) Int. Cl.: A61K 8/97, A61P 17/14, A61Q 5/00, A61Q 7/00, A61K 36/898

(54) **HAIR GROWTH AGENT / HAIR TONIC**
HAARWUCHSMITTEL / HAARTONIKUM
AGENT POUR LA POUSSE DE CHEVEUX/TONIQUE CAPILLAIRE

(30) Priority: 09.02.2011 JP 2011026187
(43) Date of publication of application: 18.12.2013
(73) Proprietor: Kawano Mericlone Co., Ltd., Mima-shi, Tokushima 779-3604 (JP)
(72) Inventor: KAWANO,Michio, Tokushima 7793604 (JP)
(74) Representative: Hewson, Timothy John
(86) International application number: PCT/JP2011/079341
(87) International publication number: WO 2012/108104

(56) References cited:
- WO-A1-2011/093769
- JP-A- 2 279 618
- JP-A- 63 145 216
- JP-A- 2002 114 646
- JP-A- 2002 114 646
- JP-A- 2002 205 933
- JP-A- 2002 205 933
- JP-A- 2005 179 219
- DATABASE GNPD [Online] MINTEL; 1 August 2009 (2009-08-01), White Sands Hair Care: "The Cure 24/7 Hair Cell Renewal", XP002751642, Database accession no. 1167723
- DATABASE GNPD [Online] MINTEL; 1 March 2007 (2007-03-01), Ted Gibson & Co.: "Daily Conditioner with Wild Orchid Extract", XP002751643, Database accession no. 683478
- DATABASE WPI Week 199101 1 January 1991 (1991-01-01) Thomson Scientific, London, GB; AN 1991-003124 XP002751644, & JP H02 279618 A (ICHIMARU PHARCOS INC) 15 November 1990 (1990-11-15)
- DAIZABURO YOKOYAMA ET AL: "Current and prospective trends of hair restorers", FUREGURANSU JANARU - FRAGRANCE JOURNAL, FUREGURANSU JANARUSHA, TOKYO, JP, vol. 25, no. 2, 1 February 1997 (1997-02-01), pages 48-55, XP008169743, ISSN: 0288-9803
- DAIZABURO YOKOYAMA ET AL.: 'Current and prospective trends of hair restorers' FRAGRANCE JOURNAL vol. 25, no. 2, February 1997, pages 48 - 55, XP008169743

## Description

### Technical Field

The present invention relates to a hair growth and fostering agent having prominent hair growth and fostering effects and anti-canities effects without causing side effects in the living body.

### Background Art

Conventionally, various studies and developments have been made on hair growth and fostering agents, and chemically synthesized substances containing components such as hormonal agents, blood flow-promoting agents, and cell-activating agents are provided. However, there have been problems that although the majority of these hair growth and fostering agents have excellent hair generation and growth and fostering effects, they also cause side effects. Further, such a hair growth and fostering agent that can be expected to have anti-canities effects has not yet been obtained.

With an aim to achieve hair growth and fostering effects while improving safety, the present inventor has proposed a hair growth and fostering agent prepared by extracting an active ingredient present in natural plants, instead of using chemically synthesized materials. That is, the present inventor has proposed a hair growth and fostering agent, comprising an extract of the pseudobulb of Great Flower Marie Laurencin, which is a plant belonging to the genus Cymbidium of the family Orchidaceae, as an active ingredient in Patent Literature 1 (Japanese Patent No. 3513475).

### Citation List

### Patent Literature

Patent Literature 1: Japanese Patent No. 3513475

### Summary of Invention

### Technical Problem

The hair growth and fostering agent described in Patent Literature 1 was highly safe since it contained a natural plant component as an active ingredient, and also achieved dramatically improved hair growth and fostering effects compared to prior products. However, on average, it requires a period of approximately six months to obtain the hair generation and growth effects after initiation of use. Although the above time period has been shortened compared to before, there is a demand for improving the hair growth and fostering effects for an even more immediate effect. Further, if a hair growth and fostering agent having anti-canities effects, namely, a hair growth and fostering agent capable of turning gray hair back to black hair, becomes available, such a hair growth and fostering agent is desirable for users.

In view of the foregoing, an object of the present invention is to provide a hair growth and fostering agent that is not only capable of further improving the hair growth and fostering effects, but also effective against canities.

### Solution to Problem

The inventor of the present application strove to achieve the aforementioned object. As a result, he has found that an extract obtained from the whole plant of Great Flower Marie Laurencin, not from only the pseudobulb, belonging to the genus Cymbidium of the family Orchidaceae has excellent hair growth and fostering effects as well as anti-canities effects with immediate actions, thereby leading to the completion of the present invention.

Plants belonging to the genus Cymbidium are monocotyledons of the family Orchidaceae, which are distributed and grow naturally mainly in the temperate and tropical regions of Asia. The plants have peculiar pseudobulbs, and there are approximately 70 species of Cymbidium plants. Further, breeding has been undertaken in plants belonging to the genus Cymbidium, and a large number of breeds are now registered under the Plant Variety Protection and Seed Act of Japan. As a representative variety of plants belonging to the genus Cymbidium, including native species, cym. goeringii, cym. kanran, cym. sinense, cym. ensifolium, cym. pumilum, Cym. Great Flower "Marie Laurencin", Cym Rose Wine "Shinseiki", Cym. Great Flower "Ballerina", Cym. Lucky Flower "Anmitsuhime", Cym. Great Katy "Mariko", Cym. Lucky Gloria "Aguri", Cym. Lucky Gloria "Fukunokami", Seaside "Saya", Cym. Satin Doll "Golden Yellow", Cym. Great Katy "Little Laurencin", Angelico "Fukumusume", and the like are known. Among native species, some are used as medicines or foods in regions such as China, Southeast Asia, and Australia.

As described above, there are an extremely large number of plants belonging to the genus Cymbidium of the family Orchidaceae. Among those plants, a variety registered under the name of Great Flower Marie Laurencin (Variety Registration No. 2841) under the Plant Variety Protection and Seed Act of Japan is used since it has particularly excellent hair growth and fostering effects as well as anti-canities effects with immediate actions.

The extract of a plant of Great Flower Marie Laurencin belonging to the genus Cymbidium of the family Orchidaceae is an extract obtained by using the whole plant of Great Flower Marie Laurencin belonging to the genus Cymbidium of the family Orchidaceae as the extraction raw material. The whole plant refers to all the parts of a plant combining the aerial and underground parts such as the flowers, leaves, flower stalks, pseudobulbs, and roots. It is preferable to use the whole plant of Great Flower Marie Laurencin belonging to the genus Cymbidium as the extraction raw material at the flowering time, especially at the peak flowering time; however, the timing may not be limited to the flowering time.

For the adjustment of the extract of the whole plant of Great Flower Marie Laurencin belonging to the genus Cymbidium of the family Orchidaceae, any of the physical extraction method, in which the whole plant of Great Flower Marie Laurencin belonging to the genus Cymbidium of the family Orchidaceae is compressed directly or after being shredded into pieces of appropriate size or pulverized by a grinder, the solvent extraction method, and the vacuum distillation method can be adopted, and extracts thereby obtained with various kinds of solvents, their dilutions, concentrates, or dried powders are used as the extract. Examples of the extraction solvent include a hydrophilic solvent such as water, methanol, ethanol, propylene glycol, 1,3-butylene glycol, glycerin, and acetone, and a mixture of two or more kinds thereof may also be used. Further, the extract to be prepared may be either a liquid or a solid.

For example, when ethanol extraction is employed, a method for producing the extract of the whole plant of Great Flower Marie Laurencin belonging to the genus Cymbidium of the family Orchidaceae is adopted such that the whole plant is immersed directly or after being shredded into pieces of appropriate size or pulverized by a grinder in a 50% aqueous solution of ethanol at a weight ratio of 2 : 7 (whole plant :50% aqueous solution of ethanol) and extraction operation is carried out with occasional stirring, and the residue is filtered off. Further, when hot water extraction is employed, the extract of the whole plant of Great Flower Marie Laurencin belonging to the genus Cymbidium of the family Orchidaceae can be produced by adding 100 ml of purified water to 5 g of the pulverized plant obtained similarly to the above, followed by thermal extraction in a boiling water bath for approximately two hours, and then adding an equivalent amount of 95% ethanol, and after leaving the resulting mixture to stand, filtering off the residue. It should be noted that the extraction method is not limited to those described above.

The extracts described above may be used in the hair growth and fostering agent directly or after arbitrarily adjusting by, for example, drying, concentrating, diluting, or mixing with other components according to the application form.

Also, the amount of the extract to be added to the hair growth and fostering agent ranges preferably from 0.1 to 30% by weight, desirably from 1.0 to 20% by weight. When the content of the extract was less than 0.1% by weight or more than 30% by weight, in either case, preferable hair growth, hair fostering and anti-canities effects could not be obtained. The most preferable added amount is 5.0 to 15% by weight, at which amount the best hair growth, hair fostering, and anti-canities effects were exhibited.

As described above, pharmaceutical products, quasi drugs, or cosmetics can be prepared by blending the extract obtained from the whole plant of Great Flower Marie Laurencin belonging to the genus Cymbidium of the family Orchidaceae with an aim to obtain such functional effects as hair growth, hair fostering, and anti-canities effects.

As the dosage form of the hair growth and fostering agent of the present invention, an externally applied agent is preferable, and it is preferably provided in the form of embrocations such as ointments, lotions, tonics, sprays, suspensions, and emulsions. As more specific usage, for example, the hair growth and fostering agent of the present invention can be used in various forms such as hair revitalizing agents, hair tonics, hair lotions, hair creams, shampoos, hair rinses, hair treatments, and hair colors.

To the hair growth and fostering agent of the present invention, moisturizing agents, surfactants, dyes, fragrances, enzymes, hormones, vitamins, ultraviolet ray absorbers, ultraviolet ray shielding agents, solvents, stabilizers, plasticizers, lubricants, solubilizers, reducing agents, buffers, sweeteners, bases, volatilization aids, adsorbents, taste masking agents, synergists, binders, suspending agents, antioxidants, gloss agents, coating agents, humectants, cooling agents, softeners, emulsifiers, excipients, antiseptic agents, preservatives, and the like can be added according to an ordinary method so long as the effect of the present invention is not impaired.

### Advantageous Effects of Invention

The present invention can provide a hair growth and fostering agent having hair generation, hair growth, and anti-canities effects with excellent immediate actions by including the extract obtained from the whole plant of Great Flower Marie Laurencin belonging to the genus Cymbidium of the family Orchidaceae as an active ingredient.

### Brief Description of Drawings

[Figure 1] Figure 1 is a photograph showing the condition in the course of usage in a test user A in Study Example 2.
[Figure 2] Figure 2 is a photograph showing the condition approximately 10 months after Figure 1.
[Figure 3] Figure 3 is a photograph showing the condition of a test user B at the beginning of use in Study Example 2.
[Figure 4] Figure 4 is a photograph showing the condition approximately 1 month after Figure 3.
[Figure 5] Figure 5 is a photograph showing the condition of a test user C at the beginning of use in Study Example 2.
[Figure 6] Figure 6 is a photograph showing the condition approximately 4 months after Figure 5.
[Figure 7] Figure 7 is a photograph showing the condition of a test user D at the beginning of use in Study Example 2.
[Figure 8] Figure 8 is a photograph showing the condition approximately 6 months after Figure 7.
[Figure 9] Figure 9 is a photograph showing the condition of a test user E at the beginning of use in Study Example 2.
[Figure 10] Figure 10 is a photograph showing the condition approximately 1 month after Figure 9.

### Description of Embodiments

The present inventor conducted intensive studies on plant extracts having the hair growth and fostering effects. As a result, he has found that excellent hair growth, hair fostering, and anti-canities effects can be obtained when the extract obtained from the whole plant of Great Flower Marie Laurencin belonging to the genus Cymbidium of the family Orchidaceae, but not the extract of the pseudobulb, is contained. The present hair growth and fostering agent has achieved the best immediate action ever, and was confirmed by a variety of tests to exert high hair growth, hair fostering, and anti-canities effects in a period of as short as approximately one month. Therefore, the extract obtained from the whole plant of Great Flower Marie Laurencin belonging to the genus Cymbidium of the family Orchidaceae can be used as an active ingredient of a hair growth and fostering agent and an anti-canities agent.

### Examples

### <Preparation of the extract of the whole plant of Great Flower Marie Laurencin belonging to the genus Cymbidium of the family Orchidaceae>

The whole plant of blooming Great Flower Marie Laurencin (Variety Registration No. 2841), which is a plant belonging to the genus Cymbidium, is shredded. The shredded plant is then added to and immersed in a 50% aqueous solution of ethanol at a weight ratio of 2 : 7 (whole plant : 50% aqueous solution of ethanol) to carry out extraction for one month at room temperature. Subsequently, the residue was filtered off and the resulting ethanol extract was used as the extract of the whole plant of Great Flower Marie Laurencin belonging to the genus Cymbidium of the family Orchidaceae.

### <Formulation Example: Hair tonic>

This Formulation Example is a formulation example of a hair tonic, and its composition is as shown below. Formulation component % by weight The aforementioned extract of the whole plant of Great Flower Marie Laurencin belonging to the genus Cymbidium of the family

| | |
|---|---|
| Orchidaceae | 10.5% by weight |
| Ethanol | 47.05% by weight |
| Cooling agent (1-menthol) | 0.30% by weight |
| Antiseptic agent(Photosensitizer 301) | 0.002% by weight |

| | |
|---|---|
| Ginger root extract | 0.10% by weight |
| Humectant | 2.652% by weight |
| Common water | 10.0% by weight |
| Purified water | Balance |

Also, examples of the humectant include, but are not limited to, sugar having moisturizing actions such as maltitol, a Rehmannia Chinensis root extract, a Tilia Cordata flower extract, a seaweed extract, and a liquid rice extract hydrolysate. These components can be blended singly or in combination. Humectants have moisturizing actions, and thus can impart appropriate moisture to the scalp and hair.

### <Comparative Example: Hair tonic>

First of all, the pseudobulb of blooming Great Flower Marie Laurencin (Variety Registration No. 2841), which is a plant belonging to the genus Cymbidium, is shredded. The shredded pseudobulb is then added to and immersed in a 50% aqueous solution of ethanol at a weight ratio of 2 : 7 (whole plant : 50% aqueous solution of ethanol) to carry out extraction for one month at room temperature. Subsequently, the residue was filtered off and the resulting ethanol extract was used as the extract of the pseudobulb of a plant belonging to the genus Cymbidium of the family Orchidaceae. Using the extract thus obtained, a hair tonic of Comparative Example was formulated. The composition of the hair tonic of Comparative Example only differs from that of the hair tonic of Formulation Example shown above in that the "extract of the whole plant of Great Flower Marie Laurencin belonging to the genus Cymbidium of the family Orchidaceae, 10.5% by weight" is replaced by the "extract of the pseudobulb of a plant belonging to the genus Cymbidium of the family Orchidaceae, 10.5% by weight", while the rest of the composition remains the same. In light of this, explanation is omitted.

Hereinbelow, the following studies were conducted in order to confirm the effect of each of Formulation Example (extract of whole plant) and Comparative Example (extract of only pseudobulb).

### <Study Example 1>

Twenty seven males and females were assigned to subjects and used the hair tonic prepared according to Formulation Example, and their feedback is summarized in Table 1 below. Also, 20 males and females were assigned to subjects and used the hair tonic prepared according to Comparative Example, and their feedback is summarized in Table 2. As to the study method, the subjects were instructed to apply and spread the aforementioned hair tonic on the head in a normal usage mode and thoroughly massage the head, and perform the above operation once or twice a day.

**[Table 1]**

| No. | Sex and Age | Before use | After use | | |
|---|---|---|---|---|---|
| | | | Time between initiation of use and manifestation of effects | Hair growth and fostering effects | Anti-can ities effects |
| Test user 1 | Male, 67 | Almost entirely gray head of fine, fragile hair | Approx. 1 month | Hair became thick and vitalized. | Hair on the back of the head started to turn back to black. After considerable improvement, hair generally turned back to black. |
| Test user 2 | Male, 57 | Bald on top | 2 to 3 months after initiation of use | Hair became so thick and vitalized overall that it was mistaken for a wig. Downy hair started to and continued to regrow on the once-bald top of the head. | |
| Test user 3 | Male, 64 | Generally thin | 1 to 2 months | Hair thinning alleviated overall and hair became vitalized and thickened. | Back of the head started to turn back to black. |
| Test user 4 | Male, 64 | Bald on top | 1 to 2 months | Hair started to regrow on top of the head, followed by rapid regrowth. | |
| Test user 5 | Male, 46 | | | Hair texture rapidly improved with rtoticeable hair regrowth in the front of the head. Hair was volumized overall. | |
| Test user 6 | Male, 63 | | 1 to 2 months | Hair became thick and vitalized with no more hair loss. People noticed the restoration of hair. | Canities alleviated and started to turn back to black. |
| Test user 7 | Male, 63 | | I to 2 months | Hair became thick and vitalized and volumized overall. | Hair on temples considerably improved. |
| Test user 8 | Male, 42 | Becoming noticeably thin | 1 to 2 months | Hair improves while rapidly becoming thick and vitalized. | A fist-sized chunk of gray hair was noticeable in the front of the head but people pointed out that it became unnoticeable. |
| Test user 9 | Male, 64 | Hair was thinning generally. Particularly concerned about partial baldness on the side of the head | 1 to 2 months | Hair was rapidly improving, relieving him from concern. | People commented that gray hair was generally turning back to black. |
| Test user 10 | Male, 63 | | 1 to 2 months | Hair became resilient and thick and volumized overall. | Gray hair turned back to black. |
| Test user 11 1 | Male, 63 | Hair was thinning generally. | I to 2 months | Hair became resilient and thick and coarse to touch. | Canities was also bering alleviated. |
| Test user 12 | Female, 56 | | Approx. 2 months | Hair loss was reduced while hair became resilient and shiny. Hairstyling can be done with a hair dryer and hands, relieving her from hair-related concerns. | |
| Test user 13 | Female, 37 | Had lost hair from the forehead to the top of the head due to radiotherapy | 1 month | Hair became strong and hair loss was also visibly reduced. | Graying hair was turning back to black. |
| Test user 14 | Female, 58 | Concerned about hair falling out during shampooing and brushing | 1 month | Hair loss was reduced. She also likes slick feel to fingers after use. | |
| Test user 15 | Female, 51 | Feels the weight and growth of hair approx. 1.5 to 2 months after haircut | | Had her hair cut approx. 1 month after use, and she started to feel the weight and growth of hair approx. 1 week after haircut. | |
| Test user 16 | Female, 57 | | A few days | Hair loss was considerably reduced in a few days after use. Combing became easy and hair was volumized, seemingly vitalized. Although having sensitive skin, the product does not cause rush and is mild to the skin. | |
| Test user 17 | Female, 52 | | 50 days | Hair loss was reduced and hair became shinier than before. She was once annoyed with itchiness but is less annoyed now. | |
| Test user 18 | Female, 47 | Flat due to fine hair | | Gained light, airy hair with reduced hair loss. Family commented that her hair was volumized. | |
| Test user 19 | Female, 51 | | 1 month | The product was mild to the scalp and hair became moistened and shiny with reduced hair loss. | |
| Test user 20 | Female, 57 | Hair on top of the head is fine and lacks resilience. | 1 month | Hair is gradually becoming resilient. | |
| Test user 21 | Female, 58 | | 40 days | Dandruff and itchiness are gone and hair became resilient and volumized. Besides that, hair grew faster. | |
| Test user 22 | Female, 53 | Concerned about limp hair | 1 months | Hair became resilient and firm. | |
| Test user 23 | Female, 58 | | 1.5 months | Relieved from concern about hair loss, and itchiness was also reduced. | |
| Test user 24 | Female, 60 | | 2 months | | Becoming less concerned about gray hair on the side of the head, which used to annoy her. |
| Test user 25 | Female, 38 | | 2 months | Hair gained an improved touch. | Once gray hair was turning to brown, making gray hair less noticeable. |
| Test user 26 | Female, 61 | Concerned about thinning on top of the head | 2.5 months | People commented that hair was volumized. | Becoming less concerned about gray hair on the hair line. |
| Test user 27 | Female, 34 | | 1.5 months | Hair became shiny. | Gray hair was also reduced. |

**[Table 2]**

| No. | Sex and Age | Before use | After use | | |
|---|---|---|---|---|---|
| | | | Time between initiation of use and manifestation of effects | Hair growth and fostering effects | Anti-canities effects |
| Test user 28 | Male, 57 | | Half a year | Hair on the front part seems to be increasing. | |
| Test user 29 | Male, 60 | | Half a year | Hair on top of the head started to grow in thickness. | |
| Test user 30 | Male, 63 | | Half a year | Downy hair gradually regrew on the back of the head. | |
| Test user 31 | Male, 64 | | Half a year | The product is non-sticky and feels fresh, and is also effective. | |
| Test user 32 | Male, 64 | | Half a year | Hair seems to become firm. Once short hair grew longer and seems to be increasing. | |
| Test user 33 | Male, 67 | | Half a year | Hair remains dandruff-free and non-itchy, and hair started to regrow in once-hairless areas. | |
| Test user 34 | Male, 87 | Bald on top | 7, 8 months | Short downy hair started to regrow in the back of the head, which is now 4 cm long. | |
| Test user 35 | Male | | Half a year | Downy hair slightly increased on the hairline, and hair was volumized overall. | |
| Test user 36 | Male, 60 | | 5, 6 months | Hair loss was reduced. | |
| Test user 37 | Male, 68 | | Half a year | Downy hair is growing on the once completely bald head. | |
| Test user 38 | Female, 70 | Thin hair | 7 months | Once limp hair was volumized, enabling her to go out without a hat. | |
| Test user 39 | Female | Fine, thin hair | Half a year | Hair became firm with reduced hair loss. | |
| Test user 40 | Female, 78 | | Half a year | Downy hair started to regrow on the sides of the forehead, where hair was thinning. | Gray hair almost disappeared. |
| Test user 41 | Female, 84 | | Half a year | | Hair turned back to black. |
| Test user 42 | Female, 73 | | Half a year | Hair started to increase. | |
| Test user 43 | Female, 70 | | 7 months | Became less concerned about thin hair on top of the head. | |
| Test user 44 | Female, 77 | | 7 months | Short hair started to regrow. | |
| Test user 46 | Female, 73 | | Half a year | Fell the product was effective. | |
| Test user 47 | Female, 60 | | Half a year | Hairdresser noticed improved hair texture and downy hair started to regrow on the front of the head. | |
| Test user 48 | Female, 81 | | Half a year | Hair started to gradually increase. | |

As is apparent from the above results, the time period until the test users felt any sort of effect was as short as 1 to 2 months with Formulation Example containing the extract of the whole plant of Great Flower Marie Laurencin belonging to the genus Cymbidium of the family Orchidaceae. This was much quicker compared to before, and almost all the test users obtained the hair growth and fostering effects. The newly grown hair was not limited to downy hair and thick hair also started to regrow. Hair gained resilience and firmness and hair loss was also reduced.

It was further found that many test users also felt profound anti-canities effects with Formulation Example containing the extract of the whole plant of Great Flower Marie Laurencin belonging to the genus Cymbidium of the family Orchidaceae. With regard to this anti-canities effect, although the mechanisms are unknown, many test users commented that it was such that existing hair turned back to black gradually from the root, rather than newly growing hair turning to black gradually from the root, revealing that canities were alleviated in a short period of time.

In contrast, the test monitors obtained the hair generation and growth effects with Comparative Example containing the extract of the pseudobulb of a plant belonging to the genus Cymbidium of the family Orchidaceae only after approximately half a year of use. Moreover, remarkable effects have not been obtained with Comparative Example with only two test users experiencing the anti-canities effects.

As described above, it was confirmed that the hair growth and fostering agent containing the extract of the whole plant of Great Flower Marie Laurencin belonging to the genus Cymbidium of the family Orchidaceae according to the present invention was effective in terms of both the hair growth and fostering effects and anti-canities effects.

### < Study Example 2>

Subsequently, five males ranging from their 40's to 70's were assigned to subjects. The changes in their hair resulting from the use of the hair tonic adjusted according to the aforementioned Formulation Example were recorded photographically and shown in Figures 1 to 10. As to the study method, the subjects were instructed to apply and spread the aforementioned hair tonic on the head in a normal usage mode and thoroughly massage the head, and perform the above operation once or twice a day.

The test user A started to use the hair tonic of Formulation Example on October 25, 2009. Thereafter, he noticed that the back of the head was turning back to black, and had his hair photographed on December 13, 2009, which was 48 days after initiation of use. The photograph is shown in Figure 1. That is, he experienced the anti-canities effect in a duration of use of approximately 1.5 months. Approximately 10 months later, another photograph was taken on October 2, 2010, which is shown in Figure 2. Comparing Figure 1 with Figure 2, it is found that the color of the hair generally turned back to black with continuous use, showing a continuous anti-canities effect.

The test user B had more gray hair than black hair according to the condition as shown in the photograph of Figure 3, which was taken at the beginning of use on October 25, 2010. However, according to the condition as shown in the photograph of Figure 4, which was taken approximately 1 month later on November 27, 2010, gray hair almost completely disappeared, revealing that canities were alleviated in as short as one month.

### Moreover, hair has gained resilience and firmness.

Compared to the condition as shown in the photograph of Figure 5, which was taken at the beginning of use on July 22, 2010, the test user C achieved hair growth, hair fostering, and anti-canities effects with the use of the hair tonic of Formulation Example. As of November 15, 2010, which was approximately four months later, the anti-canities effects and hair growth and fostering effects were prominently exhibited as shown in the photograph of Figure 6.

Despite the fact that the test user D is relatively elderly (73 years old), compared to the condition as shown in the photograph of Figure 7, which was taken at the beginning of use on May 30, 2010, canities has been alleviated particularly on top of the head, and moreover, hair has increased according to the condition as shown in the photograph of Figure 8, which was taken approximately six months later on November 23, 2010. That is, the hair generation, hair growth, and anti-canities effects were obtained even in a relatively elderly individual, in whom the revitalization of hair cannot be expected very much.

In the test user E, it is found that, compared to the condition as shown in the photograph of Figure 9, which was taken at the beginning of use on October 7, 2010, canities has been alleviated while new hair has grown, and moreover, hair has increased according to the condition of the photograph of Figure 10, which was taken only approximately one month later on November 15, 2010.

As described above, the hair growth and fostering agent of the present invention successfully produced the hair growth and fostering effects and anti-canities effects in a short period of time.

### Industrial Applicability

The present invention can be usefully employed as a hair growth and fostering agent-having prominent hair growth and fostering effects as well as anti-canities effects without causing side effects in the living body.

## Claims

1. A hair growth and fostering agent having an anti-canities effect, comprising, as an active ingredient, an extract obtained from a whole plant of Great Flower Marie Laurencin, which is a plant belonging to a genus Cymbidium of the family Orchidaceae.

2. The hair growth and fostering agent according to claim 1, wherein the hair growth and fostering agent comprises the extract in an amount of 0.1 to 30% by weight.

3. The hair growth and fostering agent-according to claim 1, wherein the hair growth and fostering agent comprises the extract in an amount of 1.0 to 20% by weight.

4. The hair growth and fostering agent according to claim 1, wherein the hair growth and fostering agent comprises the extract in an amount of 5.0 to 15% by weight.

## Patentansprüche

1. Haarwuchs- und Haarpflegemittel mit einem Anti-Canities-Effekt, das, als aktiven Bestandteil, einen Extrakt umfasst, der von einer vollständigen Pflanze der Großen Blume Marie Laurencin erhalten wurde, welche eine Pflanze darstellt, die zu einer Gattung Cymbidium der Orchideen-Familie zählt.

2. Haarwuchs- und Haarpflegemittel nach Anspruch 1, wobei das Haarwuchs- und Haarpflegemittel den Extrakt in einer Menge von 0,1 bis 30 Gewichts-% umfasst.

3. Haarwuchs- und Haarpflegemittel nach Anspruch 1, wobei das Haarwuchs- und Haarpflegemittel den Extrakt in einer Menge von 1,0 bis 20 Gewichts-% umfasst.

4. Haarwuchs- und Haarpflegemittel nach Anspruch 1, wobei das Haarwuchs- und Haarpflegemittel den Extrakt in einer Menge von 5,0 bis 15 Gewichts-% umfasst.

## Revendications

1. Agent pour la pousse et le renforcement des cheveux ayant un effet anti-canitie, comprenant, en tant que substance active, un extrait obtenu à partir d'une plante entière de grande fleur de Marie Laurencin, qui est une plante appartenant à un genre Cymbidium de la famille des Orchidaceae.

2. Agent pour la pousse et le renforcement des cheveux selon la revendication 1, dans lequel l'agent pour la pousse et le renforcement des cheveux comprend l'extrait en une quantité de 0,1 à 30 % en poids.

3. Agent pour la pousse et le renforcement des cheveux selon la revendication 1, dans lequel l'agent pour la pousse et le renforcement des cheveux comprend l'extrait en une quantité de 1,0 à 20 % en poids.

4. Agent pour la pousse et le renforcement des cheveux selon la revendication 1, dans lequel l'agent pour la pousse et le renforcement des cheveux comprend l'extrait en une quantité de 5,0 à 15 % en poids.
